## Europäisches Patentamt
## European Patent Office
## Office européen des brevets

(19)

(11) Veröffentlichungsnummer: **0 130 532**
**B1**

(12) **EUROPÄISCHE PATENTSCHRIFT**

(45) Veröffentlichungstag der Patentschrift: **22.08.90**

(21) Anmeldenummer: **84107323.2**

(22) Anmeldetag: **26.06.84**

(51) Int. Cl.⁵: **B 01 J 27/12,** B 01 J 37/26,
B 01 J 29/26, B 01 J 23/26,
C 07 C 17/20, C 07 C 17/24

(54) **Verfahren zur Herstellung von Katalysatoren für Fluorierungs- und/oder Dismutierungsreaktionen von Halogenkohlenwasserstoffen.**

(30) Priorität: **29.06.83 DE 3323374**

(43) Veröffentlichungstag der Anmeldung:
**09.01.85 Patentblatt 85/02**

(45) Bekanntmachung des Hinweises auf die
Patenterteilung:
**22.08.90 Patentblatt 90/34**

(84) Benannte Vertragsstaaten:
**BE CH DE FR GB IT LI NL**

(56) Entgegenhaltungen:
**EP-A-0 036 123**
**CA-A-1 124 265**
**DE-A-2 032 098**
**US-A-3 043 889**
**US-A-4 056 490**

**CHEMICAL ABSTRACTS, Band 98, Nr. 25, 20.
Juni 1983, Seite 521, Zusammenfassung Nr.
215170q, Columbus, Ohio, US; & JP-A-57 197
232 (ASAHI GLASS CO. LTD) 03-12-1982**

(73) Patentinhaber: **HOECHST
AKTIENGESELLSCHAFT
Postfach 80 03 20
D-6230 Frankfurt am Main 80 (DE)**

(72) Erfinder: **Müller, Thomas, Dr.
Tempelhofer Weg 3
D-6380 Bad Homburg (DE)**
Erfinder: **Siegemund, Günter, Dr.
Frankfurter Strasse 21
D-6238 Hofheim am Taunus (DE)**

Courier Press, Leamington Spa, England.

EP 0 130 532 B1

# Beschreibung

Die Erfindung betrifft ein Verfahren zur Herstellung, von aktiv und selektiv wirkenden chromhaltigen Katalysatoren. Die Katalysatoren lassen sich verwenden für die Fluorierung von halogenierten Kohlenwasserstoffen, insbesondere aliphatischen Chlorkohlenwasserstoffen, mit Fluorwasserstoff in der Gasphase oder für die Dismutierung von fluorhaltigen Kohlenwasserstoffen.

Die Herstellung chromhaltiger Katalysatoren kann nach dem Stand der Technik auf mehreren Wegen erfolgen. So ist es möglich, einen aktiven Katalysator zu erhalten, wenn man ein Oxidhydrat des dreiwertigen Chroms einer thermischen Behandlung und/oder einer Fluorierung mittels Fluorwasserstoff unterwirft. Oxidhydrate des dreiwertigen Chroms [$Cr_2O_3 \times H_2O$] ist die umfassende Bezeichnung für die verschiedenen Hydratstufen des Chrom-(III)-hydroxids (s. Friche-Hütting: "Hydroxide und Oxidhydrate", Leipzig 1937 S. 252 bis 268).

Chromoxidhydrate kann man erhalten, wenn man eine sechswertige Chromverbindung mit einem Reduktionsmittel wie Alkohol (US—A3 258 500) umsetzt und das ausgefallene Hydroxid durch Erhitzen auf ca. 500°C aktiviert. Der erhaltene Katalysator besteht in der Hauptsache aus $Cr_2O_3$.

Chromoxidhydrate, die durch Reduktion von sechswertigen Chromverbindungen in der Borsäureschmelze erhalten werden (Guignets-Grün) eignen sich als Ausgangsmaterial für Fluorierungskatalysatoren, sofern sie durch aufwendige Waschungen von borhaltigen Beimengungen befreit worden sind.

Ein Nachteil der aufgezeigten Herstellungswege ist der Einsatz von Chrom(VI)-Verbindungen, die canzerogen und toxisch sind und daher einen hohen Aufwand für technische Sicherheitsmaßnahmen erfordern.

Man kann auch Chromoxidhydrate aus einer wäßrigen Lösung einer dreiwertigen Chromverbindung mit einem alkalischen Fällungsmittel wie Ammoniumhydroxid ausfällen und durch Trocknen einen Chromoxid-Katalysator gewinnen (DE—A14 43 382). Chromoxide-γ-Hydrat kann auch durch thermische Behandlung (US—A3 426 009) und/oder Fluorierung mit Fluorwasserstoff (DE—A12 52 182) in eine katalytisch aktive Form gebracht werden.

Von Nachteil ist es, daß gefälltes Chromoxidhydrat mit großen Mengen Wasser gewaschen werden muß, um es von löslichen (chromfreien) Nebenprodukten zu befreien, die sonst die katalytischen Eigenschaften beeinträchtigen würden (Chim. Ind. (Milano) *64* (1982) S. 137, Tabelle 2). Die erforderlichen Waschvorgänge bedeuten einen erheblichen Aufwand an Zeit und Material. Bei diesem Verfahren fällt ein Produkt mit hohem Cr-Gehalt an.

Von Nachteil ist ferner die geringe mechanische Stabilität von Formkörpern aus Chromoxidhydraten, die auf diesem Wege hergestellt wurden (DD—PS 117 444). Um dieses Problem zu lösen, hat man bereits geeignete Chromverbindung auf mechanisch-stabile Träger aufgebracht. Chrom kann hierbei als Chromoxidhydrat durch Tränken des Trägers mit einer Chrom(VI)-Verbindung und anschließender Reduktion erzeugt werden (DE—A14 43 197). Ebenso kann man einen Träger mit einer Chrom(III)-salz-lösung tränken und anschließend eine Fällung von Chromhydroxid durch Alkali durchführen (DE—B12 10 783).

Auf einen Träger aufgebrachte Oxidhydrate können durch thermische Behandlung und/oder Fluorierung mit Fluorwasserstoff in eine katalytisch aktive Form überführt werden. Geeignete Trägermaterialien für Chromoxidhydrat sind z.B. Aluminiumoxid, Aluminiumfluorid, Magnesiumfluorid (DE—A14 43 382) oder Aktivkohle.

Sofern bei der Herstellung von Trägerkatalysatoren mit Chrom(VI)-Verbindungen (nebst nachfolgender Reduktion) gearbeitet wird, so ist hier neben der Giftigkeit des Ausgangsprodukts auch der vergleichsweise hohe Aufwand zur Beseitigung löslicher Nebenprodukte von Nachteil. Wegen ihres niedrigen Chromgehaltes zeigen diese Trägerkatalysatoren schließlich eine geringere Aktivität als reine Chromoxidhydrate.

So führt beispielsweise unter gewissen Bedingungen ein Katalysator aus aktiviertem "Chromoxidhydrat auf Aktivkohle" mit 14 Gew.-% Chromgehalt bei der Reaktion von Hexachlorethan mit Fluorwasserstoff nur zu einer Ausbeute von 1% Pentafluorchlorethan (DD—C—119 033, Beispiel 10). Ein trägerfreier Katalysator aus Chromoxidhydrat führt jedoch bei der gleichen Reaktion unter ähnlichen Bedingungen zu einer Ausbeute an Pentafluorethan von 68% (DD—PS 117 963, Beispiel 4).

Es ist bekannte, durch Zusatz von Bindemitteln die mechanische Festigkeit von Katalysator-Formkörpern zu erhöhen. So ist es möglich durch gemeinsame alkalische Fällung eines Chrom(III)-Salzes und eines Magnesiumsalzes die mechanische Festigkeit zu verbessern (JA—A 74 43 922). Der benutzte Katalysator enthält z.B. 2 bis 30 Gew.-% Mg. Chrom liegt als Oxidhydrat vor.

Durch Zusatz von 15% Magnesiumoxychloridbinder (Sorels Zement) zu hexagonalem CrOOH kann die mechanische Festigkeit des Katalysators erhöht werden (US—PS 3 978 145).

Bei der Herstellung von Chromoxidhydrat durch Reduktion von Chromtrioxid in Gegenwart von Magnesiumfluorid ergeben sich Katalysatoren mit verbesserten Eigenschaften. Der Magnesiumfluorid-Gehalt beträgt höchstens 50% (US—A 3 992 325).

Aus US—A—3.043.889 sind Massen bekannt, die $MgF_2$ und, 1 bis ca. 60% $Cr_2O_3$ enthalten. Sie werden hergestellt durch gemeinsames Ausfällen von Chromhydroxid und Magnesiumfluorid. Ihr Einsatzzweck ist die katalytische Chlorwasserstoffabspaltung von Trichlortrifluorethan unter Bildung von Chlortrifluorethen. Dabei wird kein Fluorwasserstoff eingesetzt.

Aus JP—A 57.197.232 ist ein Verfahren zur Herstellung von Katalysatoren bekannt, bei dem eine wäßrige Lösung der Nitrate von Aluminium, Chrom und Magnesium mit Ammoniak behandelt wird. Die ausgefallenen Hydroxide werden isoliert, getrocknet, bei 450°C calciniert und dann mit Fluorwasserstoff behandelt. Nachteilig ist dabei, daß das Chromoxid seine Reaktionsfähigkeit gegenüber Fluorwasserstoff bei Temperaturen oberhalb 400°C verliert. Die Katalysatoren werden eingesetzt für den Chlor/Fluor-Austausch von Halogenalkanen.

Es bestand somit die Aufgabe, ein Verfahren zur Herstellung der Katalysatoren für Fluorierungs- und/oder Dismutierungsreaktionen von halogenierten Kohlenwasserstoffen in der Gasphase zu finden, das den Einsatz von giftigen Chrom (VI)-Verbindungen vermeidet und keine aufwendigen Waschvorgänge erfordert. Der nach dem erfindungsgemäßen Verfahren hergestellte Katalysator soll ohne zusätzliche Maßnahmen mechanisch sehr stabil sein und eine hohe Aktivität zeigen.

Das erfindungsgemäße Verfahren zur Herstellung der eines Chrom und Magnesium enthaltenden Katalysators für Fluorierungsreaktionen löst diese Aufgabe. Es beruht auf dem Ausfällen von Chrom(III)-Hydroxid. Es ist dadurch gekennzeichnet, daß man 1 Mol eines wasserlöslichen Chrom(III)-Salzes mit mindestens 1,5 Mol Magnesiumhydroxid oder Magnesiumoxid in Gegenwart von Wasser umsetzt, man gegebenenfalls durch Eindampfen die Reaktionsmischung in einen Teig überführt, der Chromhydroxid und ein Magnesiumsalz enthält, man den Teig trocknet und bei Temperaturen von 20 bis 500°C mit Fluorwasserstoff behandelt.

Der Teig kann ferner in Abhängigkeit von dem angewandten Magnesiumüberschuß noch MgO bzw. $Mg(OH)_2$ enthalten.

Der so gewonnene chromhaltige Katalysator soll mindestens 55 Gew.-% Magnesiumfluorid und von 0,5 bis 29 Gew.-% Chrom enthalten, wobei das Atomverhältnis Magnesium/Chrom 1,5 bis 50 beträgt.

Als Chrom(III)-Verbindungen können wasserfreie und vorteilhafterweise auch die hydratisierten Salze des dreiwertigen Chroms eingesetzt werden, die leicht zugänglich und kommerziell erhältlich sind. Verwendbar sind beispielsweise Chromsulfat und Chromfluorid, vorzugsweise aber Chrom(III)-chlorid und Chrom(III)-nitrat.

Das eingesetzte Magnesiumoxid darf nicht geglüht sein, sondern muß noch mit schwach sauren Verbindungen reagieren können. Da bei der Herstellung des Katalysators kein Auswaschvorgang eingeschaltet wird, ist das Molverhältnis Magnesium/Chrom der Einsatzkomponenten dem der fertigen Katalysators gleich. Es ist vorteilhaft, das Atomverhältnis Chrom/Magnesium von 0,001 bis 0,66, vorzugsweise 0,04 bis 0,5, anzusetzten. Überraschenderweise werden schon sehr aktive Katalysatoren erhalten, wenn der Chromgehalt nur 1 Gew.-% beträgt. Bevorzugt sind Chromgehalte von 1 bis 20%. In den meisten Fällen werden 12 bis 24 Mol Magnesiumoxid oder Magnesiumhydroxid pro Mol des Chrom(III)-Salzes eingesetzt, was Atomverhältnissen Chrom/Magnesium von 0,04 bis 0,08 im fertigen Katalysator entspricht.

Die angewandte Wassermenge für die Reaktion ist nicht kritisch; die Wassermenge muß jedoch ausreichen, damit die Masse zumindest durch einen Kneter verarbeitbar ist. Je höher die eingesetzte Wassermenge, umso mehr Wasser muß schließlich verdampft werden.

Zur Herstellung der erfindungsgemäßen Katalysatoren kann man einmal die Chromverbindung als wäßrige Lösung zu trockenem Magnesiumoxid (oder -hydroxid) geben und die resultierende Reaktionsmischung zu einer Paste verkneten.

Man kann auch das vorgelegte Magnesiumoxid mit Wasser anteigen, die Chromverbindung trocken zugeben und die Reaktionsmischung verkneten. Das Verkneten erfolgt vorteilhafterweise mit Maschinen, die in der Verfahrenstechnik üblicherweise zum Vermischen pastöser Stoffe dienen (z.B. Vertikal-Kneter oder Duplex-Kneter).

Bei der Herstellung der erfindungsgemäßen Katalysatoren wird eine Paste erhalten, die ohne Waschen getrocknet wird. Sie ist direkt zur Herstellung von Formkörpern geeignet. Von Vorteil ist es, daß die Formkörper mit den üblichen verfahrenstechnischen Maßnahmen, wie. z.B. Pelletisieren, Extrudieren oder Granulieren hergestellt werden können. Die Katalysatoren sind geeignet zum Einsatz im Festbett-, Fließbett- und Wirbelschichtreaktoren.

Nach der Formgebung erfolgt die Trocknung der Kontaktförmlinge, die zu mechanischsehr stabilen Katalysatorkörpern führt. Die Trocknung kann sowohl bei Raumtemperatur als auch bei erhöhter Temperatur erfolgen. Zweckmäßigerweise wird eine Trocknungstemperatur von 50° bis 150°C, vorzugsweise 70°C bis 120°C gewählt, um die Trocknungszeit zu verkürzen. Die Trocknung kann sowohl unter Normaldruck als auch unter Vakuum erfolgen.

Die Trocknung soll nicht bei Temperaturen über 400°C erfolgen, da sonst das Chromoxid seine Reaktionsfähigkeit gegenüber Fluorwasserstoff verliert und kein Chromoxidfluorid mehr gebildet wird (US—A 32 58 500, Spalte 4).

Die Behandlung mit Fluorwasserstoff erfolgt zweckmäßigerweise bei einer Temperatur, bei der flüchtige Reaktionsprodukte ($H_2O$, HF) nicht kondensieren. Vorteilhaft sind Temperaturen von 100 bis 400°C, vorzugsweise 120 bis 220°C. Die eingesetzte Menge an Fluorwasserstoff ist nicht kritisch. Man erhält schon wirksame Katalysatoren, wenn pro Mol eingesetzter Metallverbindung (Chromsalz, Magnesiumoxid) das Kontaktmaterial mit 2 Mol Fluorwasserstoff behandelt wird. Auch noch höhere Fluorwasserstoff-Mengen sind möglich; ihr Einsatz ist nur durch wirtschaftliche Erwägungen begrenzt. Die Fluorierungszeit kann in weiten Grenzen gewählt werden; bevorzugt sind 0,5 bis 10 Stunden. Um das entstehende Wasser schneller zu entfernen und unerwünschte Temperaturspitzen zu vermeiden, kann man HF durch ein Intertgas (z.B. $N_2$ oder Luft) verdünnen.

Die Behandlung mit Fluorwasserstoff zur Aktivierung des Kontaktmaterials erfolgt zweckmäßigerweise in der gleichen Apparatur, in der später der Katalysator zur Fluorierung von Halogenkohlenwasserstoffen eingesetzt wird.

Die nach dem erfindungsgemäßen Verfahren hergestellten Katalysatoren können unmittelbar zur Durchführung von Fluorierungs- und/oder Dismutierungsreaktionen mit Halogenalkanen eingesetzt werden. Eine Aktivierung des Katalysators durch Kalzinieren ist nicht erforderlich.

Man kann auch Katalysatoren nahezu gleicher Bruttozusammensetzung herstellen, indem man pulverförmiges Chromoxidhydrat mit pulverförmigem Magnesiumfluorid vermischt und mit Fluorwasserstoff behandelt. Dieses Gemisch hat jedoch deutlich schlechtere katalytische und mechanische Eigenschaften als der erfindungsgemäß hergestellte Katalysator.

Die Erfindung wird durch die Beispiele näher erläutert:

Beispiel 1

1 Gew.-Teil CrCl$_3$· 6 H$_2$O wird in 10 Gew.-Teilen Wasser aufgelöst. Diese Lösung wird zu einer Mischung von 3,85 Gew.-Teilen Magnesiumoxid und 1,23 Gew.-Teilen Graphit gegeben und die dabei entstehende pastöse Masse innig verknetet.

Anschließend wird das pastöse Reaktionsprodukt zu Würfelförmlingen (0,5 cm Kantenlänge) granuliert und 16 Stunden bei 100°C getrocknet.

0,33 l (Schüttvolumen) der getrockneten Kontaktkörper (=200 g) werden in einem Rohr aus Nickel oder VA-Stahl mit 5 cm lichter Weite und 100 cm Länge bei 200°C mit 5 mol Fluorwasserstoff behandelt. Die Dauer der Fluorwasserstoffbehandlung beträgt ca. 3 Stunden. Dabei wird HF mit N$_2$ verdünnt. Der erhaltene Fluorierungskatalysator weist einen Chromgehalt von 1,6 Gew.-% auf.

Beispiel 2

1 Gew.-Teil Cr(NO$_3$)$_3$ · 9 H$_2$O wird in 5 Gew.-Teilen Wasser gelöst. Diese Lösung wird zu einer Mischung von 2,5 Gew.-Teilen Magnesiumoxid und 1,2 Gew.-Teilen Graphit gegeben. Die Weiterverarbeitung und Fluorwasserstoffbehandlung erfolgt gemäß Beispiel 1. Der beispielsgemäße Fluorierungskatalysator weist einen Chromgehalt von 2,3 Gew.-% auf.

Beispiel 3

1 Gew.-Teil Cr(NO$_3$)$_3$· 9 H$_2$O wird in 2,78 Gew.-Teilen Wasser aufgelöst. Diese Lösung wird zu einer Mischung von 1,39 Gew.-Teilen Magnesiumoxid und 1 Gew.-Teil Graphit gegeben. Die Weiterverarbeitung und Fluorwasserstoffbehandlung erfolgt gemäß Beispiel 1. Der beispielsgemäße Fluorierungskatalysator enthält 2,7 Gew.-% Chrom.

Beispiel 4

1 Gew.-Teil Cr(NO$_3$)$_3$ · 9 H$_2$O wird in 1,39 Gew.-Teilen Wasser aufgelöst. Diese Lösung wird zu einer Mischung von 0,69 Gew.-Teilen Magnesiumoxid und 0,68 Gew.-Teilen Graphit gegeben. Die Weiterverarbeitung und Fluorwasserstoffbehandlung erfolgt gemäß Beispiel 1. Der beispielsgemäße Fluorierungskatalysator enthält 4.3 Gew.-% Chrom.

Beispiel 5

1 Gew.-Teil CrF$_3$ · 4 H$_2$O, 1,67 Gew.-Teile MgO und 0,93 Gew.-Teile Graphit werden trocken vermischt und mit 5,47 Gew.-Teilen Wasser angeteigt und verknetet. Die Weiterverarbeitung und Fluorwasserstoffbehandlung erfolgt gemäß Beispiel 1. Der beispielsgemäße Katalysator enthält 7,3 Gew.-% Chrom.

Die folgenden Beispiele zeigen an Hand von Fluorierungs- und Dismutierungsreaktionen die Leistungsfähigkeit der erfindungsgemäßen Katalysatoren.

Beispiel 6

570 g Tetrachlorkohlenstoff und 120 g Fluorwasserstoff werden innerhalb von 3 Stunden in gasförmigen Zustand über 330 cm$^3$ des nach Beispiel 1 hergestellten Fluorierungskatalysators geleitet, der mittels eines elektrischen Widerstands auf einer Temperatur von 200°C gehalten wird.

Der Reaktor besteht aus dem gleichen Rohr, das schon zur Fluorwasserstoffbehandlung bei der Herstellung des Katalysators eingesetzt wurde.

Die den Reaktor verlassenden gasförmigen Reaktionsprodukte werden einer mit Wasser gefüllten Waschvorlage zugeführt, in der die zu analysierenden Säuregemische aufgefangen werden.

Die Werte des Fluorwasserstoffumsatzes werden in den betreffenden Beispielen mittels Titration des Waschwassers bestimmt. Die erhaltenen Werte werden zusätzlich durch eine Fluoridspezifische Elektrode potentiometrisch kontroliert. Die gasförmigen, nicht wasserlöslichen, Reaktionsprodukte werden gaschromatographisch analysiert.

Der HF-Umsatz für den nach Beispiel 1 hergestellten Fluorierungskatalysators beträgt.

99,5%

Das Reaktionsabgas zeigt folgende Zusammensetzung:

| | |
|---|---|
| CCl$_4$: | 11,8% |
| CFCl$_3$: | 21,6% |
| CF$_2$Cl$_2$: | 65,1% |
| CF$_3$Cl: | 1,5% |

Beispiel 7

Der gemäß Beispiel 2 hergestellte Fluorierungskatalysator wird in derselben Versuchsanordnung gemäß Beispiel 6 zur Fluorierung von Tetrachlorkohlenstoff mit Fluorwasserstoff eingesetzt. Die Reaktortemperatur beträgt 140°C. Der HF-Umsatz beträgt:

99,7%

Das Reaktionsabgas zeigt folgende Zusammensetzung:

CCl$_4$:       9,3%
CFCl$_3$:     25,1%
CF$_2$Cl$_2$:  61,6%
CF$_3$Cl:      3,8%

Beispiel 8

Der gemäß Beispiel 3 hergestellte Fluorierungskatalysator wird in derselben Versuchsanordnung gemäß Beispiel 6 zur Fluorierung von Tetrachlorkohlenstoff mit Fluorwasserstoff eingesetzt. Die Reaktortemperatur beträgt 180°C.

Der HF-Umsatz beträgt:

99,6%

Das Reaktionsabgas zeigt folgende Zusammensetzung:

CCl$_4$:      11,1%
CFCl$_3$:     19,2%
CF$_2$Cl$_2$:  54,8%
CF$_3$Cl:     14,3%

Beispiel 9

Der gemäß Beispiel 4 hergestellte Fluorierungskatalysator wird in derselben Versuchsanordnung gemäß Beispiel 6 zur Fluorierung von Tetrachlorkohlenstoff mit Fluorwasserstoff eingesetzt. Die Reaktortemperatur beträgt 140°C.

Der HF-Umsatz beträgt:

99,8%

Das Reaktionsabgas zeigt folgende Zusammensetzung:

CCl$_4$:       8,7%
CFCl$_3$:     15,7%
CF$_2$Cl$_2$:  70,6%
CF$_3$Cl:      4,6%

Beispiel 10

Der gemäß Beispiel 5 hergestellte Fluorierungskatalysator wird in derselben Versuchsanordnung gemäß Beispiel 6 zur Fluorierung von Tetrachlorkohlenstoff mit Fluorwasserstoff eingesetzt. Die Reaktortemperatur beträgt 200°C.

Der HF-Umsatz beträgt:

99,5%

Das Reaktionsabgas zeigt folgende Zusammensetzung:

CCl$_4$:       8,9%
CFCl$_3$:     13,2%
CF$_2$Cl$_2$:  75,4%
CF$_3$Cl:      3,4%

Beispiel 11

In derselben Versuchsapparatur gemäß Beispiel 6 werden 450 g Chloroform und 120 g Fluorwasserstoff innerhalb von 3 Stunden in gasförmigen Zustand über 0,33 l (Schüttvolumen) des gemäß Beispiel 2 hergestellten Fluorierungskatalysators geleitet, der bei einer Temperatur von 207°C gehalten wird.

Der HF-Umsatz beträgt:

98,9%

Das Reaktionsabgas zeigte folgende Zusammensetzung:

CHCl$_3$:     22,3%
CHFCl$_2$:     4,7%
CHF$_2$Cl:     5,6%
CHF$_3$:      67,4%

Beispiel 12

In derselben Versuchsapparatus gemäß Beispiel 6 werden 60 g Tetrafluordichlorethan innerhalb einer Stunde über 0,33 l (Schüttvolumen) des gemäß Beispiel 2 hergestellten Katalysators geleitet, der bei einer Temperatur von 300°C gehalten wird. Das den Reaktor verlassende Produktgas zeigt folgende Zusammensetzung:

C$_2$F$_2$Cl$_4$:     5,8%
C$_3$F$_3$Cl$_3$:    14,8%
C$_2$F$_4$Cl$_2$:    59,4%
C$_2$F$_5$Cl:        20,1%

Beispiel 13

In Versuchsapparatur gemäß Beispiel 12 wird der gemäß Beispiel 4 hergestellte Katalysator zur Dismutierung von Tetrafluordichlorethan bei 300°C eingesetzt. Das den Reaktor verlassende Produktgas zeigt folgende Zusammensetzung:

C$_2$F$_2$Cl$_4$:     5,5%
C$_2$F$_2$Cl$_3$:    16,5%
C$_2$F$_4$Cl$_2$:    50,5%
C$_2$F$_5$Cl:        27,5%

**Patentansprüche**

1. Verfahren zur Herstellung eines Chrom und Magnesium enthaltenden Katalysators für Fluorierungsreaktionen durch Aufällen von Chrom(III)-hydroxid, dadurch gekennzeichnet, daß man 1 Mol eines wasserlöslichen Chrom(III)-salzes mit mindestens 1,5 Mol Magnesiumhydroxyd oder Magnesiumoxid in Gegenwart von Wasser umsetzt, man—gegebenenfalls durch Eindampfen—die Reaktionsmischung in einen Teig überführt, der Chromhydroxid und ein Magnesiumsalz enthält, man den Teig trocknet und bei Temperaturen von 20 bis 500°C mit Fluorwasserstoff behandelt.

2. Verfahren nach Anspruch 1, dadurch gekennzeichnet, daß der Teig verformt wird, bevor er getrocknet wird.

3. Verfahren nach Anspruch 1, dadurch gekennzeichnet, daß 12 bis 24 Mol Magnesiumoxid oder Magnesiumhydroxid pro Mol des Chrom(III)-salzes eingesetzt werden.

4. Verfahren nach Anspruch 1, dadurch gekennzeichnet, das Chrom(III)-chlorid oder -nitrat eingesetzt werden.

**Revendications**

1. Procédé pour la préparation d'un catalyseur contenant du chrome et du magnésium pour réactions de fluoration par précipitation d'hydroxyde de chrome (III) caractérisé en ce qu'on fait réagir 1 mole d'un sel soluble dans l'eau de chrome(III) avec au moins 1,5 moles d'hydroxyde de magnésium ou d'oxyde de magnésium en présence d'eau, qu'on convertit, éventuellement par évaporation, le mélange réactionnel en une pâte contenant de l'hydroxyde de chrome et un sel de magnésium, qu'on sèche la pâte et qu'on la traite à l'aide d'acide fluorhydrique à des températures de 20 à 500°C.

2. Procédé selon la revendication 1, caractérisé en ce que la pâte est déformée avant séchage.

3. Procédé selon la revendication 1, caractérisé en ce qu'on utilise de 12 à 24 moles d'oxyde de magnésium ou d'hydroxyde de magnésium par mole du sel de chrome (III).

4. Procédé selon la revendication 1, caractérisé en ce qu'on utilise le chlorure ou le nitrate de chrome (III).

**Claims**

1. A process for preparing a chromium- and magnesium-containing catalyst for fluorination reactions by precipitating chromium (III) hydroxide, which comprises reacting 1 mole of a water-soluble chromium (III) salt with at least 1.5 moles of magnesium hydroxide or magnesium oxide in the presence of water, converting the reaction mixture—if necessary by evaporation—into a paste which contains chromium hydroxide and a magnesium salt, drying the paste and treating it with hydrogen fluoride at temperatures of 20 to 500°C.

2. The process as claimed in claim 1, wherein the paste is molded before it is dried.

3. The process as claimed in claim 1, wherein 12 to 24 moles of magnesium oxide or magnesium hydroxide are used per mole of chromium (III) salt.

4. The process as claimed in claim 1, wherein chromium (III) chloride or nitrate is used.